# EUROPEAN PATENT SPECIFICATION

(11) **EP 2 900 267 B1**
(45) Date of publication and mention of the grant of the patent: **15.05.2019**
(21) Application number: 13841842.1
(22) Date of filing: 27.09.2013
(51) Int. Cl.: G01N 33/68, A61K 31/4985, A61K 31/505, A61K 31/519, A61K 31/53

(54) **CYSTATIN C AND GALECTIN-3 AS BIOMARKERS FOR PULMONARY ARTERIAL HYPERTENSION**
CYSTATIN-C UND GALECTIN-3 ALS BIOMARKER FÜR PULMONALE ARTERIELLE HYPERTONIE
CYSTATINE C ET GALECTINE 3 COMME BIOMARQUEURS DE L'HYPERTENSION ARTÉRIELLE PULMONAIRE

(30) Priority: 27.09.2012 US 201261706411 P; 10.05.2013 US 201361822111 P; 20.09.2013 US 201361880459 P
(43) Date of publication of application: 05.08.2015
(73) Proprietor: Siemens Healthcare Diagnostics Inc., Tarrytown, NY 10591 (US)
(72) Inventor: LASALVIA, Luis, White Plains, NY 10601 (US); FENSTER, Brett, Parker, CO 80134 (US); SCHROEDER, Joyce, Boulder, CO 80306 (US); BUCKNER, J., Kern, Littleton, CO 80120 (US)
(74) Representative: Schweitzer, Klaus
(86) International application number: PCT/US2013/062262
(87) International publication number: WO 2014/052803

(56) References cited:
- US-A1- 2009 197 922
- US-A1- 2009 269 779
- US-A1- 2010 143 954
- IX ET AL: "Cystatin C, Left Ventricular Hypertrophy, and Diastolic Dysfunction: Data From the Heart and Soul Study", JOURNAL OF CARDIAC FAILURE, CHURCHILL LIVINGSTONE, NAPERVILLE, IL, US, vol. 12, no. 8, 1 October 2006 (2006-10-01), pages 601-607, XP005686404, ISSN: 1071-9164
- PEREZ VINICIO A DE JESUS ET AL: "Diagnosis and management of pulmonary hypertension associated with left ventricular diastolic dysfunction", PULMONARY CIRCULATION 2012 APR-JUN, vol. 2, no. 2, April 2012 (2012-04), pages 163-169, XP002757933, ISSN: 2045-8940
- UMESH C SHARMA ET AL: "Galectin-3 marks activated macrophages in failure-prone hypertrophied hearts and contributes to cardiac dysfunction", CIRCULATION, LIPPINCOTT WILLIAMS & WILKINS, US, vol. 110, no. 19, 9 November 2004 (2004-11-09), pages 3121-3128, XP008154272, ISSN: 0009-7322, DOI: 10.1161/01.CIR.0000147181.65298.4D [retrieved on 2004-11-01]
- BUCKLEY MITCHELL S ET AL: "Phosphodiesterase-5 inhibitors in management of pulmonary hypertension: safety, tolerability, and efficacy.", DRUG, HEALTHCARE AND PATIENT SAFETY 2010, vol. 2, 2010, pages 151-161, XP002757935, ISSN: 1179-1365
- SHAH ET AL.: 'Galectin-3, cardiac structure and function, and long-term mortality in patients with acutely decompensated heart failure' EUROPEAN JOURNAL OF HEART FAILURE vol. 12, 2010, pages 826 - 832, XP055125266
- P EREZ-CALVO ET AL.: 'Differential prognostic utility of NTproBNP and Cystatin C in patients with acute exacerbation of chronic pulmonary disease.' JRSM SHORT REP. vol. 1, no. 5, 21 October 2010, pages 44; PAGES 1 - 9, XP055252296

## Description

### RELATED APPLICATIONS

This application claims the benefit of provisional patent application Nos. 61/706,411 filed September 27, 2012; 61/822,111 filed May 10, 2013 and 61/880,459 filed September 20, 2013.

### FIELD OF THE INVENTION

The present invention relates to the use of biomarkers for diseases and conditions of the heart for diagnosis and treatment regimens, especially for the diagnosis and treatment of pulmonary arterial hypertension (PAH).

### BACKGROUND

Pulmonary arterial hypertension (PAH) is a progressive disorder characterized by increased pulmonary vascular resistance resulting in right ventricular dysfunction and ultimately heart failure. Accordingly, indices of right ventricular dysfunction are the most important prognostic indicators in pulmonary arterial hypertension. IX ET AL discloses in the study "Cysratin C, Left Venticular Hypertrophy and Diagnostic Dysfunction: Data from the Heart and Soul Study", JOURNAL OF CARDIAC FAILURE, CHURCHILL LIVINGSTONE, NAPERVILLE, IL, US, vol.12, no.8, 1 October 2006, pages 601-607, XP005686404, ISSN: 1071-9164 a study in which the association of serum cystatin C with left ventricular hypertrophy (LVH) diastolic dysfunction, and systolic dysfunction was examined in a cross-sectional study among 818 outpatients with coronary artery diseases. According to this study it is hypothesized that participants in the highest cystatin C quartile were more likely to have LVH and diastolic dysfunction compared with patients in the lowest cystatin C quartile.

UMESH C SHARMA ET AL: "Galectin-3 marks activated macrophages in failure-prone hypertrophied hearts and contributes to cardiac dysfunction", CIRCULATION; LIPPINCOTT WILLIAMS & WILKINS, US, vol. 110, no. 19, 9. November 2004, pages 3121-3128; XP008154272, ISSN: 0009-7322, DOI: 10.1161/01.CIR.0000147181.65298.4Dis directed to study showing that an early increase of 3-galetic expression identifies failure-prone hypertrophied hearts. PEREZ VINICIO A DE JESUS AT AL:"Diagnosis and management of pulmonary hypertension associated with left ventricular diastolic dysfunction": PULMONARY CIRCULATION 2012 APR-JUN, vol. 2, no. 2, APRIL 2012, pages 163-169, XP002757933, ISSN: 2045-8940 is directed to the diagnosis and management of pulmonary hypertension associated with left ventricular diastolic dysfunction. It is disclosed therein that pulmonary hypertension is often associated with left heart failure. Further, BUCKLEY MITCHELL S ET AL: "Phosphodiesterase-5 inhibitors in management of pulmonary hypertension: safety, tolerability and efficacy", DRUG, HEALTHCARE AND PATIENT SAFETY 2010, vol.2, 2010, pages 151-161, XP002757935, ISSN: 1179-1365 describes phosphordiesterase-5 inhibitors, in particular sildenafil, tadalafil and vardenafil for treating pulmonary arterial hypertension (PAH).

Biomarkers that reflect right ventricular distress, including brain natriuretic peptide (BNP) and its N-terminal cleavage product, NT-ProBNP, are currently used for the diagnosis and prognosis of pulmonary arterial hypertension. However, the utility of BNP and NT-ProBNP as pulmonary arterial hypertension biomarkers is limited by the false negative readings these markers provide in early pulmonary arterial hypertension, attenuated levels in obesity, and physiologic elevations with age and female gender, independent of cardiovascular status.

### SUMMARY OF THE INVENTION

The present invention provides a method of identifying a candidate for pulmonary arterial hypertension treatment, wherein the method includes providing a sample of a biological fluid from a test subject; determining the concentration of CysC and/or Gal-3 in the sample from the test subject; comparing the concentration of CysC and/or Gal-3 determined in the sample form the test subject with a normal range of CysC and/or Gal-3 concentration, respectively; determining that the level of CysC and/or Gal-3 is above the normal range of CysC and/or Gal-3 concentration and thereby identifying the test subject as a candidate for treatment of pulmonary arterial hypertension.

Also described herein is a method of diagnosis of pulmonary arterial hypertension in a test subject, wherein the method includes providing a sample of a biological fluid from a test subject; determining the level of CysC and/or Gal-3 in the sample from the test subject; comparing the level of CysC and/or Gal-3 determined in the sample form the test subject with a normal range of CysC and/or Gal-3 levels, respectively; determining that the level of CysC and/or Gal-3 is above the normal range of CysC and/or Gal-3 levels and thereby diagnosing the test subject as likely suffering from pulmonary arterial hypertension.

When levels of both CysC and Gal-3 in the sample from the test subject are above the normal range for each of these biomarkers, the elevated level of Gal-3 is a confirmatory result and provides a high degree of confidence that the test subject is suffering from pulmonary arterial hypertension.

### BRIEF DESCRIPTION OF THE FIGURES

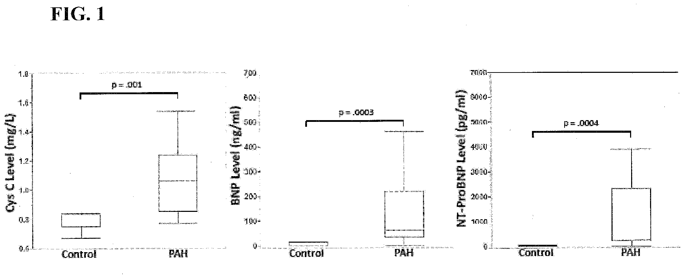

Figure 1 shows Biomarker levels in control vs. PAH subjects. Left panel: CysC, middle panel: BNP, right panel: NT-ProBNP. Box plots display the distribution of data including the minimum (bottom whisker), first quartile (bottom of rectangle), median (horizontal line within rectangle), third quartile (top of rectangle), and maximum (top whisker).

### DETAILED DESCRIPTION

Pulmonary arterial hypertension (PAH) is one of five pulmonary hypertension (PH) groups of conditions classified by the World Health Organization. In addition to the pulmonary arterial hypertension condition of Group 1, these conditions include Group 2: PH with left heart disease; Group 3: PH with lung disease and/or hypoxemia; Group 4: PH due to chronic thrombotic and/or embolic disease; and Group 5: miscellaneous conditions (including sarcoidosis, histiocytosis X, lymphangiomatosis and compression of pulmonary vessels).

PAH is a serious, progressive and life-threatening disease of the pulmonary vasculature system, characterized by profound vasoconstriction and an abnormal proliferation of smooth muscle cells in the walls of the pulmonary arteries. Severe constriction of the blood vessels in the lungs leads to very high pulmonary arterial pressures. These high pressures make it difficult for the heart to pump blood through the lungs to be oxygenated. Patients with PAH suffer from extreme shortness of breath as the heart pumps against these high pressures. Patients with PAH typically develop significant increases in pulmonary vascular resistance and sustained elevations in pulmonary artery pressure, which can ultimately lead to right ventricular failure and death. Patients diagnosed with PAH have a poor prognosis and equally compromised quality of life, with a mean life expectancy of 2 to 5 years from the time of diagnosis if untreated.

Although the non-invasive imaging assessment of PAH has traditionally focused on Doppler echocardiographic-derived pulmonary artery systolic pressure (PASP), estimated PASP has yet to demonstrate prognostic significance. Therefore, the value of cardiac imaging appears to lie in the assessment of right ventricular adaptation to PAH. RVEF, RV end diastolic volume (RVEDV), and right ventricular end systolic volume (RVESV) all predict right heart failure, clinical deterioration, and mortality. The recent development of novel imaging techniques allows for a more comprehensive approach to right ventricular assessment that may supplement traditional methodologies. The complex geometry of the right ventricle and predominantly longitudinal myofibril orientation has led to the development of speckle-tracking echocardiography, an angle-independent technique for assessing right ventricular motion. Furthermore, echocardiographic assessment of right ventricle filling pressures using a combination of tissue and pulsed-wave Doppler is a powerful predictor of cardiac events, functional status, and exercise capacity, indicating right ventricular diastolic dysfunction (RVDD) has prognostic significance in PAH. Using a same-day, multimodality imaging approach to comprehensively characterize the right ventricle, the following hypothesis was tested: That CysC is as strongly associated with right ventricle size, systolic function, strain, and right ventricular diastolic function as the traditional biomarkers, BNP and NT-ProBNP, in pulmonary arterial hypertension (PAH).

Cystatin C (CysC) is a non-glycosylated basic protein, formerly known as "gamma trace" and was first detected in urine, cerebrospinal fluid and other biological fluids from patients with renal failure. It has since been used as a highly sensitive, endogenous marker of renal filtration. However, CysC also predicts left heart failure and overall cardiovascular mortality. Furthermore, CysC may be superior to NT-ProBNP in the prediction of acute left heart failure-related mortality. Significantly and in contrast to BNP and NT-ProBNP, serum CysC levels appear to be independent of muscle mass, age, and gender, supporting the use of CysC diagnostics and monitoring as a more reliable indicator of pulmonary arterial condition.

Galectin-3 (Gal 3) is a marker of left heart failure and cardiovascular mortality due to congestive heart failure (CHF). Galectin-3 is an approximately 30Kd protein member of the lectin family, containing a carbohydrate recognition domain of about 130 amino acids. Galectin-3 is a galactose-specific lectin that binds IgE and is involved in acute inflammatory responses including neutrophil activation and adhesion, chemoattraction of monocytes and macrophages, opsonization of apoptotic neutrophils, and activation of mast cells.

Phosphodiesterase-5 inhibitors (PDE-5 inhibitors) such as sildenafil, tadalafil and vardenafil are used for treatment of pulmonary arterial hypertension (PAH): See for instance, U.S. Patent No. 8,377,933; as are endothelin-receptor antagonists such as ambrisentan, which is used in the treatment of PAH to prevent further thickening of the pulmonary vascular system. Inhibition of JAK2 has also been suggested as another therapeutic approach for the treatment of PAH; See U.S. Patent No. 8,410,173. U.S. Patent No. 8,324,247 discloses use of combined 5-HT2A and 5-HT2B receptor antagonists for the treatment of pulmonary arterial hypertension.

### ABBREVIATIONS:

CMR: Cardiac MRI
CysC: Cystatin C
BNP: Brain natriuretic peptide
NT-ProBNP: N-terminal pro brain natriuretic peptide
GFR: Glomerular filtration rate
LVEF: Left ventricular ejection fraction
LVEDV: Left ventricular end diastolic volume
LVESV: Left ventricular end systolic volume
PAH: Pulmonary arterial hypertension
PASP: Pulmonary artery systolic pressure
RV: Right ventricle
RVDD: Right ventricular diastolic dysfunction
RVEF: Right ventricular ejection fraction
RVEDV: Right ventricular end diastolic volume
RVESV: Right ventricular end systolic volume
RSVP: Right ventricular systolic pressure
TV E/e': Tricuspid valve E/e' ratio
TV E/A: Tricuspid valve E/A ratio.

In one embodiment the present invention provides a method for identifying a candidate for pulmonary arterial hypertension treatment, wherein the method includes: providing a sample of a biological fluid from a subject; determining the concentration of CysC and/or Gal-3 in the sample; comparing the concentration of CysC and/or Gal-3 with a normal range of CysC concentration and/or Gal-3 concentration, respectively; and determining that the concentration of CysC and/or Gal-3 is above the normal concentration range and thereby identifying the subject as a candidate for pulmonary arterial hypertension treatment. The biological fluid sample can be any suitable biological fluid sample. Such as for instance and without limitation: a blood, plasma or serum sample. Test subjects who are candidates for PAH treatment present with one or more of the following symptoms: chest pain, shortness of breath, fluid in the lungs and dizziness.

In another embodiment the invention provides a method for identifying a candidate for pulmonary arterial hypertension treatment, wherein the concentration of CysC in the sample is determined by forming a complex of the CysC in the sample with a CysC binding agent and determining the concentration of the complex. The concentration of the CysC in the sample with a CysC binding agent is related to the concentration of CysC in the sample. A concentration of CysC in the sample above the normal range is indicative that the subject is likely suffering from pulmonary arterial hypertension. The CysC binding agent can be any suitable CysC binding agent, such as for instance, an anti-CysC antibody, e.g. the anti-CysC antibody of the Siemens ADVIA Chemistry assay.

In still another embodiment the invention provides a method for identifying a candidate for pulmonary arterial hypertension treatment, wherein the concentration of Gal-3 in the sample is determined by forming a complex of the Gal-3 in the sample with a Gal-3 binding agent and determining the concentration of the complex. The concentration of the Gal-3in the sample with a Gal-3binding agent is related to the concentration of Gal-3in the sample. A concentration of Gal-3 in the sample above the normal range is indicative that the subject is likely suffering from pulmonary arterial hypertension. Alternatively, in a subject having an above-normal concentration of CysC in a sample of a biological fluid, a finding of a concentration of Gal-3 in the sample above the normal range is confirmation that the subject is likely suffering from pulmonary arterial hypertension. The Gal-3 binding agent can be any suitable Gal-3 binding agent, such as for instance, an anti-Gal-3 antibody, e.g. the anti-Gal-3 antibody of the Siemens ADVIA Chemistry assay.

Described herein is a method for identifying and treating a candidate for pulmonary arterial hypertension treatment, wherein the method includes: providing a sample of a biological fluid from a subject; determining the concentration of CysC and/or Gal-3 in the sample; comparing the concentration of CysC and/or Gal-3 with a normal range of CysC concentration and/or Gal-3 concentration, respectively; and determining that the concentration of CysC and/or Gal-3 is above the normal concentration range and thereby identifying the subject as a candidate for pulmonary arterial hypertension (PAH) treatment and treating the subject with a PAH therapy. The PAH therapy can be any suitable PAH therapy, such as for instance treatment with a PDE-5 inhibitor such as sildenafil, tadalafil or vardenafil; or an endothelin-receptor antagonist such as ambrisentan.

Further, a method for monitoring a treatment of pulmonary arterial hypertension in a subject is described herein, wherein the method includes: providing two or more samples of a biological fluid from a subject at different times during therapy for pulmonary arterial hypertension; determining the concentration of CysC in the samples; comparing the concentration of CysC determined in at least two if the samples; and thereby monitoring the treatment of the pulmonary arterial hypertension.

### EXAMPLES

All human trials were conducted in compliance with all Federal State and local regulatory requirements and under the approval of an Institutional Review Board (IRB).

### EXAMPLE 1: Comparison of CysC with Standard Markers for the Diagnosis and Monitoring of Pulmonary Arterial Hypertension (PAH)

CLINICAL TRIALS REGISTRATION: clinicaltrials.gov, NCTO1491646

### MATERIALS AND METHODS:

### Study Population.

The prospective study was approved by the National Jewish Health Institutional Review Board, and informed consent was obtained from all subjects. Subjects were recruited and consecutively enrolled from the National Jewish Health Cardiology Clinic. All subjects were aged 18 years or older and had no contraindications to cardiac MRI (CMR). To remove alternative causes of biomarker elevation or attenuation, subjects were excluded if they had cardiomyopathy; coronary artery disease (history of myocardial infarction; mechanical revascularization, including percutaneous coronary intervention or coronary artery bypass surgery; or coronary artery stenosis of greater than 50%); significant, i.e. above moderate valvular heart disease; advanced renal insufficiency (glomerular filtration rate of less than or equal to 30 ml/kg/1.73 M²) or advanced liver disease (cirrhosis, chronic hepatitis B or C infection, or cancer). Controls were asymptomatic, without parenchymal lung disease, pulmonary hypertension, or smoking history. Prior to enrollment, all PAH subjects had previous right-heart catheterization-proven PAH as per World Health Organization criteria as well right ventricular diastolic dysfunction (RVDD) per echocardiogram done within two weeks prior to enrollment. Biometric data including age, gender, and body mass index (BMI), as well as New York Heart Association Functional Class, were collected on the day of protocol performance.

### Doppler Echocardiography.

Data acquisition was performed with a Vivid 7 ultrasound system (General Electric Medical System, Milwaukee, WI, USA) equipped with a 3-MHz transducer. Pulsed tissue Doppler image recordings were acquired from an apical four-chamber view during a short end-expiration pause. Using pulsed-wave Doppler echocardiography, right ventricular filling parameters were obtained including early (E) and late (A) filling peak velocities (cm/s), and E/A ratio. Using pulsed tissue Doppler, lateral and septal tricuspid annulus early (E') diastolic velocities (cm/s) as well as E/E' ratio were obtained. RVDD was defined as either stage I (TV E/A < 0.8, TV E/e' > 6, and DT > 120 ms) or stage II (TV E/A = 0.8-2.1, E/e' > 6, and DT > 120 ms) as per American Society of Echocardiography guidelines. Right ventricular systolic pressure (RVSP) was estimated using the tricuspid regurgitant envelope method as described by Yock et al., Circulation. 1984; 70: 657-62.

### Right Ventricular Longitudinal Strain.

Following two-dimensional echocardiography acquisition, apical four-chamber images were imported into Syngo Velocity Vector Imaging v2.0 (Siemens Medical Solutions USA, Inc., Mountain View, California) for strain measurement. The right ventricular endocardial border was manually traced in a mid-systolic frame and automatically tracked to determine basal right ventricular free wall peak systolic longitudinal myocardial strain as previously described (Pirat et al., Am J of Card 2006, 98: 699-704).

### Cardiac MRI.

CMR was performed the same day as echocardiography. Subjects underwent CMR imaging in the supine position using a 1.5 T clinical MRI system (Magnetom Avanto, Siemens Medical Solutions, Erlangen, Germany) and eight-channel phased array cardiac coil. The cardiac short axis was determined from scout images at the level of the mid-ventricle, vertical long axis, and a horizontal long axis. The basal short-axis image plane was positioned beyond the level of the tricuspid valve plane. A cine steady-state free precession technique with retrospective gating was used to image from the base to apex during brief end-expiratory breath-holds using contiguous short-axis slices in 8 mm increments. Right ventricular and left ventricular volumetric and functional analysis was performed off-line by a blinded reader using commercially available software (Argus, Syngo MR B17, Siemens Medical Solutions, Erlangen, Germany). Right and left ventricular end-diastolic and end-systolic contours were manually traced for each slice. RVEDV and RVESV were determined according to the modified Simpson's rule (See Lorenz et al., J Cardiovasc Magn Reson 1999, 1:7-21). RVEF and cardiac index (RV CI) were calculated. The same methodology was applied for determination of left ventricular ejection fraction (LVEF), left ventricular end diastolic volume (LVEDV), and left ventricular end systolic volume (LVESV).

### Laboratory Diagnostics.

BNP, CysC, NT-ProBNP, and creatinine were collected from all subjects via phlebotomy immediately before echocardiography and CMR. Following collection and centrifugation, samples were aliquoted and stored at -20 °C using plasma-EDTA (BNP, CysC) or serum (NT-ProBNP, creatinine). Following completion of the study, all samples were sent to a core laboratory facility for analysis. Plasma BNP concentration was determined using the ADVIA Centaur immunoassay, CysC and creatinine levels were measured using ADVIA Chemistry assays, and NT-ProBNP concentration was determined using the Visa immunoassay (Siemens Healthcare Diagnostics, Tarrytown, New York). All samples were run in triplicate and averaged by a single operator who was blinded to echocardiography, CMR, and patient characteristics. Glomerular filtration rate (GFR) was calculated using the Modification of Diet in Renal Disease Study equation.

### Statistical Analysis.

Because histogram analysis with the Kolmogorov-Smirnov test failed to verify a normal distribution, the Wilcoxon rank sum test was used to compare CysC, BNP, NT-ProBNP, GFR, age, gender, and BMI between control and PAH groups. We report median with associated interquartile range for continuous data and percentages for categorical data. Spearman correlation coefficients were used to test the strength of the relationship between CysC, BNP, NT-ProBNP and markers of RV pressure, size, systolic function, and diastolic function using JMP v9.0 (SAS Institute, Cary, NC). Values of p < 0.05 were taken to be statistically significant (NS = not significant).

RESULTS: A total of twenty-four subjects were enrolled, including fourteen with PAH and ten controls. PAH was either idiopathic (N=12) or associated with connective-tissue disease (N=2). The was no difference in gender between control and PAH groups (70% vs. 79%, p = NS). Furthermore, there was no significant difference in age or BMI as shown in Table 1 below.

**Table 1: Biometric and demographic characteristics in control and PAH groups.**

| | **Control (N=10)** | **PAH (N=14)** | **p Value** |
|---|---|---|---|
| **Age (years)** | 59 (54-66) | 64 (60-70) | NS |
| **BMI (kg/m²)** | 25 (23-26) | 28 (24-31) | NS |
| **GFR (cc/min/1.73 m²)** | 75 (70-84) | 71 (48-101) | NS |
| **LVEF (%)** | 71 (66-79) | 69 (63-75) | NS |
| **LVEDV (cc)** | 96 (78-106) | 87 (73-112) | NS |
| **LVESV (cc)** | 27 (19-35) | 27 (22-46) | NS |

| | | | |
|---|---|---|---|
| Data is reported as median value with associated interquartile range in parentheses. BMI = body mass index, GFR = glomerular filtration rate, LVEF = left ventricular ejection fraction, LVEDV = left ventricular ejection diastolic volume, and LVESV = left ventricular end systolic volume. | | | |

Because of the potential for differences in renal function to confound CysC comparisons, MDRD GFR (Glomerular Filtration Rate based on the Modification of Diet in Renal Disease equation) was calculated and found to be similar between the two groups (median 75 ml/kg.1.73 M², interquartile range 71-84 ml/kg/1.73 M² in controls vs. 71 ml/kg/1.73 M², interquartile range 48-101 ml/kg/1.73 M² in PAH subjects, p = NS). Similarly, there were no significant between-group differences in markers of left ventricular morphology or function, including LVEF, LVEDV, or LVESV. Nine of the 14 PAH subjects were on targeted pharmacologic therapy on the day of protocol completion (7 sildenafil, 1 tadalafil, and 1 ambrisentan). All PAH subjects were New York Heart Association Functional Class (NYHA FC) III, and all controls were asymptomatic.

### Biomarker Levels

CysC levels were abnormally elevated and significantly higher in the PAH group compared to controls (median 1.06 mg/L, interquartile range 0.86-1.24 mg/L vs. 0.79 mg/L, interquartile range 0.75-0.83 mg/L, p .001, reference range of 0.53-0.95 mg/L) (see Figure 1). Similarly, compared to controls, subjects with PAH had higher levels of both BNP (median 3 ng/ml, interquartile range 0-15 ng/ml vs. 63 ng/ml, interquartile range 34-220 ng/ml, p = 0.0003, reference range 0-100 ng/ml) and NT-ProBNP (median 40 pg/ml, interquartile range 23-63 pg/ml vs. 462 pg/ml, interquartile range 63-2383 pg/ml, p = 0.0004, reference range 0-125 pg/ml). Consistent with the known effect of renal function on these biomarkers, both CysC and NT-ProBNP levels negatively correlated with GFR (r=-0.43, p = 0.03 and r= 0.48, p = 0.02, respectively). In addition, BNP approached a statistically significant relationship with GFR (r =-0.40, p=0.05). None of the biomarkers were correlated with age, gender, or BMI.

**TABLE 2: Baseline characteristics in right ventricular pressure, size, systolic function, and diastolic function in control vs. PAH subjects.**

| | **Control** | **PAH** | **p Value** |
|---|---|---|---|
| **RVSP (mmHg)** | 28 (22-32) | 57 (46-65) | < 0.0001 |
| **RVEF (%)** | 51 (44-55) | 35 (27-39) | 0.0005 |
| **RV Lateral Peak Strain** | -39 (-53 to -23) | -22 (-28 to -14) | 0.02 |
| **RV Lateral Peak Strain Rate (1/s)** | -3 (-4 to -1) | -1.1 (-1.8 to -0.9) | NS |
| **RVEDV (cc)** | 122 (105-171) | 192 (140-208) | 0.04 |
| **RVESV (cc)** | 59 (46-94) | 125 (75-152) | 0.004 |
| **RV Mass (g/M²)'** | 13 (11-17) | 23 (19-29) | 0.0001 |
| **TV E/e'** | 3.4 (2.5-4.1) | 5.9 (4.8-6.7) | 0.0002 |
| **TV e'** | 12.5 (10.8-15.6) | 6.3 (5.9-7.1) | <0.0001 |
| **TV E/A** | 1.7 (1.3-2.1) | 0.8 (0.7-1.1) | 0.003 |

| | | | |
|---|---|---|---|
| Data is reported as median value with associated interquartile range in parentheses. RVSP = right ventricular systolic pressure, RVEF = right ventricular ejection fraction, RVEDV = right ventricular ejection diastolic volume, RVESV = right ventricular end systolic volume, TV E/e' = tricuspid valve E to e' peak velocity ratio, TV e' = tricuspid valve e' velocity, and TV E/A = tricuspid valve E to A peak velocity ratio. | | | |

### Right Ventricular Systolic Function and Pressure

Compared with controls, the PAH group had lower RVEF, greater RVEDV and RVESV, and higher RVSP as shown in Table 2, above. CysC levels positively correlated with RVSP, RV peak lateral strain, and RV peak lateral strain rate and negatively correlated with RVEF as shown in Table 3 below.

**TABLE 3: Spearman's coefficients for correlations between biomarkers and indices of RV pressure and systolic function.**

| | **CysC** | **BNP** | **NT-ProBNP** |
|---|---|---|---|
| **RVSP** | 0.61 (<0.01) | 0.69 (<.01) | 0.74 (<0.01) |
| **RVEF** | -0.58 (<0.01) | -0.55 (<0.01) | -0.61 (<0.01) |
| **RV Lateral Peak Strain** | 0.51 (0.01) | 0.41 (0.04) | 0.49 (0.02) |
| **RV Lateral Peak Strain Rate** | 0.51 (0.01) | 0.36 (NS) | 0.45 (0.03) |

| | | | |
|---|---|---|---|
| p Values are reported in parentheses. RVSP = right ventricular systolic pressure, RVEF = right ventricular ejection fraction. | | | |

Although both BNP and NT-ProBNP were more strongly correlated with RVSP than CysC, CysC demonstrated a stronger relationship to right ventricular strain and strain rate than either BNP or NT-ProBNP, and a comparable correlation to RVEF.

### Right Ventricular Morphology and Diastolic Function

Indices of myocardial remodeling, including RVEDV, RVESV, and RV mass index, were all increased in the PAH group relative to controls (see Table 2 above). In addition, in PAH subjects, markers of right ventricular diastolic function, including TV E/e' and TV E/A, were significantly increased, and TV e' was significantly decreased, relative to controls. CysC was positively correlated with RVEDV, RVESV, right ventricular mass index, and right ventricular E/e' and negatively correlated with both TV E/A and TV e'. BNP and NT-ProBNP relationships with RVEDV, RVESV, right ventricular mass, and TV E/e' were correlated with CysC (see Tables 4 and 5 below).

**Table 4. Spearman's coefficients for correlations between biomarkers and indices of right ventricular morphology.**

| | **CysC** | **BNP** | **NT-ProBNP** |
|---|---|---|---|
| **RV Mass g/M2** | 0.67 (<0.01) | 0.70(<0.01) | 0.67(<0.01) |
| **RVESV (cc)** | 0.58 (<0.01) | 0.52 (<0.01) | 0.58 (<0.01) |
| **RVEDV (cc)** | 0.50 (.01) | 0.44 (0.03) | 0.42 (<0.04) |

| | | | |
|---|---|---|---|
| p values are noted in parentheses. RVEDV = right ventricular ejection fraction, RVESV = right ventricular end systolic volume. | | | |

**Table 5. Spearman's coefficients for correlations between biomarkers and indices of right ventricular diastolic function.**

| | **CysC** | **BNP** | **NT-ProBNP** |
|---|---|---|---|
| **TV e' (cm/s)** | -0.75 (<0.01) | -0.61 (<0.01) | -0.66 (<0.01) |
| **TV E/e'** | 0.57 (<0.01) | 0.57 (<0.01) | 0.58 (<0.01) |
| **TV E/A** | -0.50 (0.01) | -0.41 (0.04) | -0.38 (NS) |

| | | | |
|---|---|---|---|
| p Values are noted in parentheses. TV e' = tricuspid valve e' velocity, TV E/e' = tricuspid valve E to e' peak velocity ratio, and TV E/A = tricuspid valve E to A peak velocity ratio. | | | |

As shown above in Table 5 the CysC correlations with TV e' and TV E/A were stronger than correlations with BNP and NT-ProBNP.

### CysC vs. Glomerular Filtration Rate for Right ventricular Correlations

CysC is commonly held to be a sensitive indicator of renal function, but in our cohort CysC was only modestly correlated with GFR (r=-0.43, p=0.03). Although we identified significant associations between GFR and both RVEF and RV mass index, the strength of those associations were weaker than the associations between CysC and these indices (r=0.51 vs. 0.58, and 0.43 vs. 0.67, respectively). Furthermore, GFR did not predict RV strain, strain rate, RVSP, or any markers of RV diastolic function.

### EXAMPLE 2: Comparison of Gal-3 with Standard Markers for the Diagnosis and Monitoring of Pulmonary Arterial Hypertension

Using a prospective study, fifteen subjects with right heart catheterization-proven pulmonary arterial hypertension (PAH) and ten age-matched controls underwent same-day echocardiography, cardiac MRI (CMR), and phlebotomy for Galectin-3 (Gal 3) and creatinine. Cardiac magnetic resonance imaging (CMR) was used to calculate left ventricular ejection fraction (LVEF), right ventricular ejection fraction (RVEF), right ventricular end systolic volume (RVESV), and right ventricular mass. Echocardiography was used to estimate right ventricular systolic pressure (RVSP) as well as assess right ventricular diastolic function using tricuspid valve (TV) e' velocity and E/e'.

All pulmonary arterial hypertension (PAH) subjects were either idiopathic (N=12) or connective-tissue disease associated (N=3). There was no significant difference in age, gender, body mass index, estimated glomerular filtration rate, or LVEF between controls and PAH subjects. RVSP in PAH subjects was significantly elevated vs. controls (58 ± 17 mmHg vs. 26 ± 8 mmHg, p < 0.01). Gal-3 levels were significantly elevated in the PAH group when compared to controls (18 ± 5 vs. 11 ± 2, p < .01). Gal-3 levels positively correlated with RVSP (r = 0.53, p < 0.01), RVESV (r = 0.41, p < 0.04), RV mass (r = 0.47, p < 0.01) and TV E/e' (r = 0.65, p < 0.01). Gal-3 negatively correlated with RVEF (r = -0.41, p = 0.04) and TV e' (r = -0.61, p < 0.01).

This study provides evidence that Gal-3 levels are predictive of pulmonary pressure, right ventricular systolic function, diastolic function, and morphology independent of left ventricular systolic function and that Gal-3 provides a novel biomarker for diagnosis and monitoring of pulmonary arterial hypertension.

### DISCUSSION:

In the CysC prospective study, fourteen subjects with right-heart catheterization-confirmed pulmonary arterial hypertension and ten healthy controls underwent comprehensive same-day assessments that included cardiac magnetic resonance imaging (CMR), echocardiography and blood draw for the purposes of testing CysC as a viable biomarker in pulmonary arterial hypertension. The observed CysC level, commonly held to be a sensitive marker of renal function, is abnormally elevated in pulmonary arterial hypertension subjects compared to controls and that the degree of both systolic and diastolic right ventricular dysfunction, as well as right ventricular systolic pressure (RVSP), were all found to be significantly correlated with CysC levels, thus supporting the utility of CysC as a pulmonary arterial hypertension (PAH) biomarker.

Based on the strength of associations with metrics known to possess prognostic value in PAH, CysC performed similarly to the two established PAH biomarkers, BNP and NT-ProBNP. Unlike BNP and NT-ProBNP, CysC has the advantage of being age-, gender-, and muscle mass-independent for determining renal function, thus making it a potentially more attractive PAH biomarker than BNP and NT-ProBNP - both vary depending on a number of parameters unrelated to PAH. Although CysC levels are elevated in left heart failure, our population was free of significant potential confounders including left heart disease including left ventricular systolic dysfunction, ischemia, or valvular disease; thus we can be highly confident that the elevated CysC levels we observed were due to pulmonary arterial hypertension.

Renal insufficiency predicts right heart failure in pulmonary arterial hypertension. Because CysC is a sensitive indicator of renal filtration, the elevated CysC levels observed might merely reflect decreases in renal function. However, the lack of a difference in glomerular filtration rate (GFR) between pulmonary arterial hypertension subjects and controls-in the face of a significant difference between groups in CysC levels-negates this as a possibility. Moreover, GFR correlated only with right ventricular ejection fraction (RVEF) and right ventricular mass index, but CysC correlated with several right ventricular markers, again showing CysC to be a more specific marker of myocardial remodeling than renal dysfunction. This notion is supported by the fact serum CysC levels are independently associated with indices of left ventricular hypertrophy including mass, concentricity, and wall thickness as measured by CMR. Furthermore, CysC is known to inhibit elastolytic activity in myocardial extracts from hypertensive rats, indicating a role of in extracellular matrix deposition.

These results demonstrate the utility of CysC as a biomarker for pulmonary arterial hypertension. Subjects had intermediate disease severity: all were New York Heart Association (NYHA) functional class III and had moderate elevations in BNP, NT-ProBNP, and right ventricular systolic pressure (RVSP) with moderately decreased right ventricular systolic function. These findings can also apply to the rest of the pulmonary arterial hypertension disease spectrum. The present study did not address this possibility due to the fact our population had relatively-preserved renal function.

The use of echo-derived RVSP as a surrogate for invasive pulmonary hemodynamics introduces the potential for significant over- and under-estimation of pulmonary artery systolic pressure (PASP). Because the right ventricle is a highly load-dependent chamber, correlations between right ventricular imaging data and biomarker are subject to potential misinterpretation by variable loading conditions. However, in the study reported here, imaging and biomarker acquisition was performed back-to-back on the same day to minimize the potential for significant bias.

The present study is strengthened by its prospective design and multimodality-imaging approach. For right ventricular imaging, cardiac MRI, the current gold standard for right heart assessment was used. Besides employing conventional right ventricular imaging metrics (including right ventricular size and systolic function), novel, easily acquired and potentially clinically-relevant markers including right ventricular strain and diastolic function were assessed, further bolstering the relevance of the above-reported findings.

### REFERENCES

1. Benza RL, Miller DP, Gomberg-Maitland M, et al. Predicting survival in pulmonary arterial hypertension: insights from the Registry to Evaluate Early and Long-Term Pulmonary Arterial Hypertension Disease Management (REVEAL). Circulation 2010; 122:164-72.
2. Chin KM, Kim NH, Rubin LJ. The right ventricle in pulmonary hypertension. Coron Artery Dis 2005; 16: 13-18.
3. Leuchte HH, Holzapfel M, Baumgartner RA, Ding I, Neurohr C, Vogeser M, Kolbe T, Schwaiblmair M, Behr J. Clinical significance of brain natriuretic peptide in primary pulmonary hypertension. J Am Coll Cardiol 2004;43:764-770.
4. Batal O, Faulx M, Krasuski RA, Khatib OF, Hammel JP, Hussein AA, Minai OA, Dweik RA. Effect of obesity on B-type natriuretic peptide levels in patients with pulmonary arterial hypertension. Am J Cardiol. 2012, 110:909-14.
5. Rivera M, Cortés R, Salvador A, Bertomeu V, de Burgos FG, Payá R, Portolés M, Taléns-Visconti R, Martinez-Dolz L, Valero R, Sevilla B, Climent V. Obese subjects with heart failure have lower N-terminal pro brain natriuretic peptide plasma levels irrespective of aetiology. Eur J Heart Fail. 2005;7: 1168-70.
6. Mehra M R, Uber P A, Park M H et al. Obesity and suppressed B-type natriuretic peptide levels in heart failure. J Am Coll Cardiol 2004; 43: 1590-1595.
7. Raymond I, Groenning BA, Hildebrandt PR, Nilsson JC, Bauman M, Trawinski J, Pedersen F. The influence of age, sex and other variables on the plasma level of N-terminal pro BNP in a large sample of the general population. Heart 2003;89:745-751.
8. Redfield M M, Rodeheffer R J, Jacobsen S J et al. Plasma brain natriuretic peptide concentration: impact of age and gender. J Am Coll Cardiol 2002; 40: 976-982.
9. Wang T J, Larson M G, Levy D et al. Impact of age and sex on plasma natriuretic peptide levels in healthy adults. Am J Cardiol 2002; 90: 254-258.
10. Fliser D, Ritz E. Serum cystatin C concentration as a marker of renal dysfunction in the elderly. Am J Kidney Dis. 2001;37:79-83.
11. Serum cystatin C is superior to serum creatinine as a marker of kidney function: A meta analysis. American Journal of Kidney Diseases 2002, 221-226.
12. Shlipak MG, Katz R, Fried LF, Jenny NS, Stehman-Breen CO, Newman AB, Siscovick D, Psaty BM, Sarnak MJ. Cystatin-C and mortality in elderly persons with heart failure. J Am Coll Cardiol. 2005;45:268-271.
13. Ix JH, Shlipak MG, Chertow GM, Whooley MA. Association of cystatin C with mortality, cardiovascular events, and incident heart failure among persons with coronary heart disease: data from the Heart and Soul Study. Circulation. 2007;115:173-179.
14. Lassus J, Harjola VP, Sund R, Siirila-Waris K, Melin J, Peuhkurinen K, Pulkki K, Nieminen MS. Prognostic value of cystatin C in acute heart failure in relation to other markers of renal function and NT-proBNP. Eur Heart J. 2007;28:1841-1847.
15. Pérez-Calvo JI, Ruiz-Ruiz FJ, Carrasco-Sánchez FJ, Morales-Rull JL, Manzano-Fernández S, Galisteo-Almeda L, Pascual-Figal D. Prognostic value of serum cystatin C and N-terminal pro b-type natriuretic peptide in patients with acute heart failure. Eur J Intern Med. 2012(7):599-603.
16. Coll E, Botey A, Alvarez L et al. Serum cystatin C as a new marker for noninvasive estimation of glomerular filtration rate and as a marker for early renal impairment. Am J Kidney Dis 2000; 36: 29-34.
17. Tang WH, Van Lente F, Shrestha K, Troughton RG, Francis GS, Tong W, Martin MG, Borowski AG, Jasper S, Starling RC, Klein AL. Impact of Myocardial Function on Cystatin C Measurements in Chronic Systolic Heart Failure. Journal of Cardiac Failure 2008, 14: 394-399.
18. Eysmann SB, Palevsky HI, Reichek N, Hackney K, Douglas PS. Two dimensional and Doppler-echocardiographic and cardiac catheterization correlates of survival in primary pulmonary hypertension. Circulation 1989;80:353-60.
19. D'Alonzo GE, Barst RJ, Ayres SM, Bergofsky EH, Brundage BH, Detre KM, Fishman AP, Goldring RM, Groves BM, Kernis JT, et al. Survival in patients with primary pulmonary hypertension. Results from a national prospective registry. Annals of Internal Medicine. 1991(5):343-9.
20. McLaughlin VV, Presberg KW, Doyle RL, Abman SH, McCrory DC, Fortin T, Ahearn G; American College of Chest Physicians. Prognosis of pulmonary arterial hypertension: ACCP evidence-based clinical practice guidelines. Chest. 2004(126):78S-92S.
21. Rudski LG, Lai WW, Afilalo J, Hua L, Handschumacher MD, Chandrasekaran K, Solomon SD, Louie EK, Schiller NB. Guidelines for the echocardiographic assessment of the right heart in adults: a report from the American Societyof Echocardiography endorsed by the Eur. Assoc Echocardiography, a registered branch of the European Society of Cardiology, and the Canadian Society of Echocardiography. Am Soc Echocardiogr. 2010: 685-713.
22. van Wolferen SA, Marcus JT, Boonstra A, Marques KM, Bronzwaer JG, Spreeuwenberg MD, Postmus PE, Vonk-Noordegraaf A. Prognostic value of right ventricular mass, volume, and function in idiopathic pulmonary arterial hypertension. Eur Heart J. 2007 May; 28(10):1250-7.
23. Utsunomiya H, Nakatani S, Nishihira M, Kanzaki H, Kyotani S, Nakanishi N, Kihara Y, Kitakaze M. Value of estimated rt ventricular filling pressure in predicting cardiac events in chronic pulmonary arterial hypertension. J Am Soc Echocardiogr. 2009 (12):1368-74.
24. Hardegree EL, Sachdev A, Villarraga HR, Frantz RP, McGoon MD, Kushwaha SS, Hsiao JF, McCully RB, Oh JK, Pellikka PA, Kane GC. Role of serial quantitative assessment of right ventricular function by strain in pulmonary arterial hypertension. Am J Cardiol. 2013(1):143-8.
25. McLaughlin VV, Archer SL, Badesch DB, Barst RJ, Farber HW, Lindner JR, Mathier MA, McGoon MD, Park MH, Rosenson RS, Rubin LJ, Tapson VF, Varga J, Harrington RA, Anderson JL, Bates ER, Bridges CR, Eisenberg MJ, Ferrari VA, Grines CL, Hlatky MA, Jacobs AK, Kaul S, Lichtenberg RC, Lindner JR, Moliterno DJ, Mukherjee D, Pohost GM, Rosenson RS, Schofield RS, Shubrooks SJ, Stein JH, Tracy CM, Weitz HH, Wesley DJ; ACCF/AHA.ACCF/AHA 2009 expert consensus document on pulmonary hypertension: a report of the Amer ColL. Cardiol. Foundn Task Force on Expert Consensus Documents and the Amer Heart Assoc: developed in collaboration with the American College of Chest Physicians, American Thoracic Society, Inc., and the Pulmonary Hypertension Association. Circulation. 2009. 119:2250-94.
26. J Rudski LG, Lai WW, Afilalo J, Hua L, Handschumacher MD, Chandrasekaran K, Solomon SD, Louie EK, Schiller NB. Guidelines for the echocardiographic assessment of the right heart in adults: a report from the Amer Soc Echocardiography endorsed by the Eur Assoc of Echocardiography, a registered branch of the European Society of Cardiology, and the Canadian Society of Echocardiography. Am Soc Echocardiogr. 2010:685-713.
27. Yock PG, Popp RL. Noninvasive estimation of right ventricular systolic pressure by Doppler ultrasound in patients with tricuspid regurgitation. Circulation. 1984;70:657-62.
28. Bahar Pirat, Marti L. McCulloch, William A. Zoghbi, Methodist DeBakey Heart Center Evaluation of Global and Regional Right Ventricular Systolic Function in Patients with Pulmonary Hypertension Using a Novel Speckle Tracking Method. Am J of Card 2006, 98: 699-704.
29. Lorenz CH, Walker ES, Morgan VL, Klein SS, Graham TP Jr.: Normal human right and left ventricular mass, systolic function, and gender differences by cine magnetic resonance imaging. J Cardiovasc Magn Reson 1999, 1:7-21.
30. Levey AS, Coresh J, Greene T, Stevens LA, Zhang YL, Hendriksen S, Kusek JW, Van Lente F: Using standardized serum creatinine values in the modification of diet in renal disease study equation for estimating glomerular filtration rate. Ann Intern Med 2006, 145:247-254.
31. Mielniczuk LM, Chandy G, Stewart D, Contreras-Dominguez V, Haddad H, Pugliese C, Davies RA. Worsening renal function and prognosis in pulmonary hypertension patients hospitalized for right heart failure. Congest Heart Fail. 2012, 18:151-7.
32. Patel PC, Ayers CR, Murphy SA. Association of Cystatin C with left ventricular structure and function: the Dallas Heart Study. Circ Heart Fail 2009;2:98-104.
33. Cheng XW, Obata K, Kuzuya M, Izawa H, Nakamura K, Asai E, Nagasaka T, Saka M, Kimata T, Noda A, Nagata K, Jin H, Shi GP, Iguchi A, Murohara T, Yokota M. Elastolytic cathepsin induction/activation system exists in myocardium and is upregulated in hypertensive heart failure. Hypertension. 2006(5):979-87.
34. Fisher MR, Forfia PR, Chamera E, Housten-Harris T, Champion HC, Girgis RE, Corretti MC, Hassoun PM. Accuracy of Doppler echocardiography in the hemodynamic assessment of pulmonary hypertension. Am J Respir Crit Care Med. 2009;179:615-621.

## Claims

1. A method of identifying a candidate for pulmonary arterial hypertension treatment, the method comprising:
providing a sample of a biological fluid from a subject;
determining the concentration of CysC and/or Gal-3 in the sample;
comparing the concentration of CysC and/or Gal-3 in the sample with a normal range of CysC concentration and/or Gal-3 concentration, respectively;
and determining that the concentration of CysC and/or Gal-3 is above the normal range of CysC and/or Gal-3 concentration, respectively and thereby identifying the subject as a candidate for pulmonary arterial hypertension treatment.

2. The method of claim 1, wherein the subject is suffering from one or more symptoms selected from chest pain, shortness of breath, fluid in the lungs and dizziness.

3. The method of claim 1, wherein the biological fluid from the subject comprises blood, plasma or serum.

4. The method of claim 1, wherein the concentration of CysC in the sample is determined by forming a complex of the CysC in the sample with a CysC binding agent and determining the concentration of the complex.

5. The method of claim 1, wherein the concentration of Gal-3 in the sample is determined by forming a complex of the Gal-3 in the sample with a Gal-3 binding agent and determining the concentration of the complex.

6. The method of claim 1, wherein the concentration above the normal range of CysC concentration or Gal-3 concentration in the sample is indicative that the subject is likely suffering from pulmonary arterial hypertension.

7. The method of claim 1, wherein the concentration of CysC and Gal-3 in the sample are both above the normal concentration range.

8. The method of claim 1, wherein
a) the CysC concentration in the sample is above 0.83 mg/L, or
b) wherein the Gal-3 concentration in the sample is above 13 mg/L, or
c) wherein both the CysC concentration in the sample is above 0.83 mg/L and the Gal-3 concentration in the sample is above 13 mg/L
and optionally in case of c) further comprising using a therapy for treating the subject's pulmonary arterial hypertension.

9. A method of monitoring pulmonary arterial hypertension in a subject, the method comprising:
providing two or more samples of a biological fluid from a subject at different times;
determining the concentration of CysC in the samples;
comparing the concentration of CysC determined in at least two of the samples; and
thereby monitoring the pulmonary arterial hypertension.

10. The method of claim 9, further comprising
determining the concentration of Gal-3 in the samples;
comparing the concentration of Gal-3 determined in at least two of the samples; and
thereby monitoring the pulmonary arterial hypertension.

11. The method of claim 9, wherein the two or more samples are from times during a therapeutic treatment for pulmonary arterial hypertension.

12. The method of claim 10, wherein the two or more samples are from times during a therapeutic treatment for pulmonary arterial hypertension.

13. A method of identifying a subject at risk for developing pulmonary arterial hypertension treatment, the method comprising:
providing a sample of a biological fluid from a subject;
determining the concentration of CysC and/or Gal-3 in the sample;
comparing the concentration of CysC and/or Gal-3 in the sample with a normal range of CysC concentration and/or Gal-3 concentration, respectively;
and determining that the concentration of CysC and/or Gal-3 is above the normal range of CysC and/or Gal-3 concentration, respectively and thereby identifying the subject as at risk for developing pulmonary arterial hypertension.

## Patentansprüche

1. Verfahren zur Identifizierung eines Kandidaten zur Behandlung von pulmonaler arterieller Hypertonie, das umfasst:
Bereitstellen einer Probe einer biologischen Flüssigkeit von einem Probanden;
Bestimmen der Konzentration von CysC und/oder Gal-3 in der Probe;
Vergleichen der Konzentration von CysC und/oder Gal-3 in der Probe mit einem Normalbereich der CysC- bzw. Gal-3-Konzentration;
und Bestimmen, dass die Konzentration von CysC und/oder Gal-3 oberhalb des Normalbereichs der CysC- bzw. Gal-3-Konzentration liegt, und dadurch Identifizieren des Probanden als Kandidaten zur Behandlung von pulmonaler arterieller Hypertonie.

2. Verfahren nach Anspruch 1, wobei der Proband an einem oder mehreren Symptomen, ausgewählt aus Brustschmerzen, Kurzatmigkeit, Flüssigkeit in den Lungen und Schwindel, leidet.

3. Verfahren nach Anspruch 1, wobei die biologische Flüssigkeit von dem Probanden Blut, Plasma oder Serum umfasst.

4. Verfahren nach Anspruch 1, wobei die Konzentration von CysC in der Probe durch Bildung eines Komplexes des CysC in der Probe mit einem CysC-Bindungsmittel und Bestimmung der Konzentration des Komplexes bestimmt wird.

5. Verfahren nach Anspruch 1, wobei die Konzentration von Gal-3 in der Probe durch Bildung eines Komplexes des Gal-3 in der Probe mit einem Gal-3-Bindungsmittel und Bestimmung der Konzentration des Komplexes bestimmt wird.

6. Verfahren nach Anspruch 1, wobei die Konzentration oberhalb des Normalbereichs der CysC-Konzentration oder der Gal-3-Konzentration in der Probe anzeigt, dass der Proband wahrscheinlich an pulmonaler arterieller Hypertonie leidet.

7. Verfahren nach Anspruch 1, wobei die Konzentrationen von CysC und Gal-3 in der Probe beide oberhalb des normalen Konzentrationsbereichs liegen.

8. Verfahren nach Anspruch 1, wobei
a) die CysC-Konzentration in der Probe oberhalb von 0,83 mg/l liegt, oder
b) wobei die Gal-3-Konzentration in der Probe oberhalb von 13 mg/l liegt, oder
c) wobei sowohl die CysC-Konzentration in der Probe oberhalb von 0,83 mg/l liegt und die Gal-3-Konzentration in der Probe oberhalb von 13 mg/l liegt,
und wahlweise im Fall von c) ferner umfassend die Verwendung einer Therapie zur Behandlung der pulmonalen arteriellen Hypertonie des Probanden.

9. Verfahren zur Überwachung von pulmonaler arterieller Hypertonie bei einem Probanden, das umfasst:
Bereitstellen von zwei oder mehr Proben einer biologischen Flüssigkeit von einem Probanden zu verschiedenen Zeitpunkten;
Bestimmen der Konzentration von CysC in den Proben;
Vergleichen der Konzentration von CysC, die in wenigstens zwei der Proben bestimmt wurde; und
dadurch Überwachen der pulmonalen arteriellen Hypertonie.

10. Verfahren nach Anspruch 9, das ferner umfasst:
Bestimmen der Konzentration von Gal-3 in den Proben;
Vergleichen der Konzentration von Gal-3, die in wenigstens zwei der Proben bestimmt wurde; und
dadurch Überwachen der pulmonalen arteriellen Hypertonie.

11. Verfahren nach Anspruch 9, wobei die zwei oder mehr Proben von Zeitpunkten während einer therapeutischen Behandlung für pulmonale arterielle Hypertonie stammen.

12. Verfahren nach Anspruch 10, wobei die zwei oder mehr Proben von Zeitpunkten während einer therapeutischen Behandlung für pulmonale arterielle Hypertonie stammen.

13. Verfahren zum Identifizieren eines bezüglich der Entwicklung pulmonaler arterieller Hypertonie gefährdeten Probanden, das umfasst:
Bereitstellen einer Probe einer biologischen Flüssigkeit von einem Probanden;
Bestimmen der Konzentration von CysC und/oder Gal-3 in der Probe;
Vergleichen der Konzentration von CysC und/oder Gal-3 in der Probe mit einem Normalbereich der CysC-Konzentration bzw. Gal-3-Konzentration;
und Bestimmen, dass die Konzentration von CysC und/oder Gal-3 oberhalb des Normalbereichs der CysC- bzw. Gal-3-Konzentration liegt, und dadurch
Identifizieren des Probanden als gefährdet bezüglich der Entwicklung pulmonaler arterieller Hypertonie.

## Revendications

1. Procédé d'identification d'un candidat pour le traitement de l'hypertension artérielle pulmonaire, le procédé comprenant les étapes consistant à :
fournir un échantillon d'un fluide biologique provenant d'un sujet;
déterminer la concentration de CysC et/ou de Gal-3 dans l'échantillon ;
comparer la concentration de CysC et/ou de Gal-3 dans l'échantillon avec une plage normale de concentration de CysC et/ou de concentration de Gal-3, respectivement ;
et déterminer que la concentration de CysC et/ou de Gal-3 est supérieure à la plage normale de concentration de CysC et/ou de Gal-3, respectivement, et ainsi identifier le sujet comme candidat au traitement de l'hypertension artérielle pulmonaire.

2. Procédé selon la revendication 1, dans lequel le sujet souffre d'un ou plusieurs symptômes choisis parmi une douleur thoracique, un essoufflement, un liquide dans les poumons et des étourdissements.

3. Procédé selon la revendication 1, dans lequel le fluide biologique provenant du sujet comprend du sang, du plasma ou du sérum.

4. Procédé selon la revendication 1, dans lequel la concentration de CysC dans l'échantillon est déterminée en formant un complexe du CysC dans l'échantillon avec un agent liant CysC et en déterminant la concentration du complexe.

5. Procédé selon la revendication 1, dans lequel la concentration de Gal-3 dans l'échantillon est déterminée en formant un complexe du Gal-3 dans l'échantillon avec un agent liant Gal-3 et en déterminant la concentration du complexe.

6. Procédé selon la revendication 1, dans lequel la concentration au-dessus de la plage normale de concentration de CysC ou de concentration de Gal-3 dans l'échantillon est indicative que le sujet souffre probablement d'hypertension artérielle pulmonaire.

7. Procédé selon la revendication 1, dans lequel la concentration de CysC et de Gal-3 dans l'échantillon sont toutes les deux supérieures à la plage de concentration normale.

8. Procédé selon la revendication 1, dans lequel
a) la concentration de CysC dans l'échantillon est supérieure à 0,83 mg/L, ou
b) dans lequel la concentration de Gal-3 dans l'échantillon est supérieure à 13 mg/L, ou
c) dans lequel la concentration de CysC dans l'échantillon est supérieure à 0,83 mg/L et la concentration de Gal-3 dans l'échantillon est supérieure à 13 mg/L
et éventuellement dans le cas de c) comprenant en outre l'utilisation d'une thérapie pour traiter l'hypertension artérielle pulmonaire du sujet.

9. Procédé de surveillance de l'hypertension artérielle pulmonaire chez un sujet, le procédé comprenant les étapes consistant à :
fournir deux ou plusieurs échantillons d'un fluide biologique provenant d'un sujet à des moments différents ;
déterminer la concentration de CysC dans les échantillons ;
comparer la concentration de CysC déterminée dans au moins deux des échantillons ; et
surveiller ainsi l'hypertension artérielle pulmonaire.

10. Procédé selon la revendication 9, comprenant en outre les étapes consistant à :
déterminer la concentration de Gal-3 dans les échantillons ;
comparer la concentration de Gal-3 déterminée dans au moins deux des échantillons; et
surveiller ainsi l'hypertension artérielle pulmonaire.

11. Procédé selon la revendication 9, dans lequel les deux échantillons ou plus proviennent de moment pendant un traitement thérapeutique de l'hypertension artérielle pulmonaire.

12. Procédé selon la revendication 10, dans lequel les deux échantillons ou plus proviennent de moment pendant un traitement thérapeutique de l'hypertension artérielle pulmonaire.

13. Procédé d'identification d'un sujet à risque pour le développement d'un traitement de l'hypertension artérielle pulmonaire, le procédé comprenant les étapes consistant à :
fournir un échantillon d'un fluide biologique provenant d'un sujet;
déterminer la concentration de CysC et/ou de Gal-3 dans l'échantillon ;
comparer la concentration de CysC et/ou de Gal-3 dans l'échantillon avec une plage normale de concentration de CysC et/ou de concentration de Gal-3, respectivement ;
et déterminer que la concentration de CysC et/ou de Gal-3 est supérieure à la plage normale de concentration de CysC et/ou de Gal-3, respectivement, et ainsi identifier le sujet comme étant à risque de développer une hypertension artérielle pulmonaire.
